(19) Europäisches Patentamt
European Patent Office
Office européen des brevets

(11) Publication number: **0 330 468**
**A2**

(12) # EUROPEAN PATENT APPLICATION

(21) Application number: 89301772.3

(22) Date of filing: 23.02.89

(51) Int. Cl.4: **A 61 K 31/395**
A 61 K 31/40, A 61 K 31/445,
A 61 K 31/55, A 61 K 31/47,
C 07 D 211/26, C 07 D 211/70

(30) Priority: 23.02.88 GB 8804106
23.02.88 GB 8804109
23.02.88 GB 8804110
23.05.88 GB 8812173

(43) Date of publication of application:
30.08.89 Bulletin 89/35

(84) Designated Contracting States:
AT BE CH DE ES FR GB IT LI NL SE

(71) Applicant: **GLAXO GROUP LIMITED**
**Clarges House 6-12 Clarges Street**
**London W1Y 8DH (GB)**

(72) Inventor: **Hayes, Ann Gail**
**5 Sandringham Road**
**Potters Bar Hertfordshire (GB)**

(74) Representative: **Pett, Christopher Phineas et al**
**Frank B.Dehn & Co. Imperial House 15-19 Kingsway**
**London WC2B 6UZ (GB)**

The title of the invention has been amended (Guidelines for Examination in the EPO, A-III, 7.3).

(54) **Use of heterocyclic derivatives as kappa-opioid receptor agonists in the treatment of cerebral ischaemia.**

(57) The invention relates to the use of certain heterocyclic compounds which act as kappa receptor agonists in the treatment of cerebral ischaemia.

EP 0 330 468 A2

Bundesdruckerei Berlin

**Description**

**MEDICAMENTS**

This invention relates to a new medical use for a group of heterocyclic derivatives and pharmaceutical compositions containing them. In particular the invention relates to the use of certain heterocyclic derivatives which act as agonists at kappa opioid receptors in the treatment of cerebral ischaemia.

The present invention relates to the use in the treatment of cerebral ischaemia of certain heterocyclic derivatives which have been disclosed in the art as having kappa opiod receptor agonist activity.

In published Australian Patent Application No. 86/66824 there are disclosed compounds or solvates or salts thereof of formula (I) :

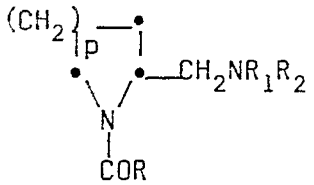

(I)

wherein

RCO represents an acyl group containing a substituted or unsubstituted carbocyclic or heterocyclic aromatic ring;

$R_1$ and $R_2$ are independently $C_{1-6}$ alkyl groups or together form a $C_{3-6}$ polymethylene or alkenylene group; and p is 1, 2, 3 or 4.

The compounds of formula (I) are described as kappa-receptor agonists which act at kappa opioid receptors in the above mentioned specification. It is stated that the compounds are useful in the treatment of pain.

Compounds which are kappa agonist receptors of use in the treatment of pain are also disclosed in published European Patent Applications No.s 232989, 228246, 260041 and 275696.

Published European Patent Application No. 232989 discloses compounds which may be represented by the formula (II)

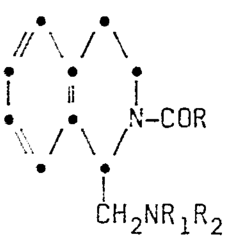

(II)

wherein R, $R_1$ and $R_2$ are as defined for formula (I).

In published European Patent No. 228246A there are disclosed compounds or solvates or salts thereof which may be represented by the formula (III)

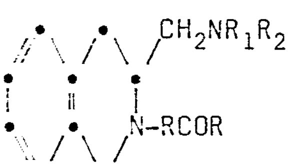

(III)

wherein

R, $R_1$ and $R_2$ are as defined for formula (I).

Published European Patent Application No. 260041 discloses compounds or solvates or salts thereof of formula (IV) :

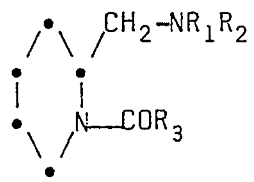

(IV)

wherein
$R_3CO$ is an acyl group in which $R_3$ represents

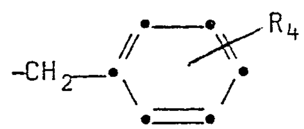

and $R_4$ is Br, $NO_2$ or $CF_3$; and $R_1$ and $R_2$ are as defined for formula (I).

These compounds fall within the scope of the group of compounds described in published European Patent Application No. 232612 but are not specifically disclosed therein. The compounds are described as having improved kappa receptor agonism properties.

In published European Patent Application No. 275696 there are disclosed compounds or salts or solvates thereof of formula (V)

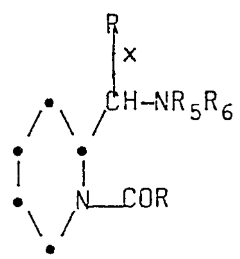

(V)

wherein
R is as defined for formula (I).
$R_5$ and $R_6$ are independently hydrogen, $C_{1-6}$alkyl, $C_{1-6}$alkenyl, $C_{1-6}$cycloalkyl or $C_{1-12}$cycloalkylalkyl or together form a $C_{2-6}$ polymethylene or $C_{2-6}$alkenylene group, optionally substituted with a heteroatom, provided that $R_5$ and $R_6$ do not both represent hydrogen; $R_x$ is $C_{1-6}$alkyl or phenyl, or $R_x$ together with $R_5$ form a $-(CH_2)_3-$or $-(CH_2)_4-$ group.

Pharmaceutically acceptable salts of compounds of formula (I) to (V) include the acid addition salts with conventional pharmaceutical acids, for example, maleic, hydrochloride, hydrobromic, phosphoric, acetic, fumaric, salicylic, citric, lactic, mandelic, tartaric, succinic, benzoic, ascorbic and methanesulphonic. The solvates may be, for example, hydrates.

Compounds which act as agonists at kappa opioid receptors and which are useful in the treatment of cerebral ischaemia have been described in the art.

N-(2-aminocycloclohexyl)benzeneacetamide compounds having kappa opioid receptors agonist activity of use in the treatment of cerebral ischaemia are disclosed in pulblished European patent application No.176309.

In published European patent application No.233793 there are disclosed certain decahydroquinoline derivatives which act at kappa opioid receptors. These compounds are described as being useful inter alia as anti-ischaemic agents.

We have now found that certain compounds which are known as Kappa agonists are useful in the treatment of cerebral ischaemia.

Thus, according to one aspect the invention provides a compound selected from compounds of formulae (I) to (V) as defined above or a pharmaceutically acceptable salt thereof for use in the treatment of cerebral ischaemia.

According to another aspect, the invention provides a method of treatment of a subject, in particular a

human subject, suffering from the effects of cerebral ischaemia which comprises administering an effective amount of a compound selected from compounds of formulae (I) to (V) as defined above or a pharmaceutically acceptable salt or solvate thereof.

The effect of the compounds of formulae (I) to (V) in protecting against neuronal damage resulting from cerebral ischaemia may be demonstrated for example using standard laboratory bilateral carotid occlusion models.

Compounds for use according to the invention may be administered as the raw material but the active ingredient is preferably presented as a pharmaceutical formulation.

In a further aspect the invention provides a compound selected from compounds of formulae (I) to (V) or a pharmaceutically acceptable salt or solvate thereof for use in the manufacture of a medicament for the treatment of cerebral ischaemia.

Pharmaceutical compositions for use according to the present invention may be formulated in conventional manner for administration by any convenient route. For example, compounds of formulae (I) to (V) may be formulated in the manner described in the above mentioned specifications. Compounds for use according to the present invention may conveniently be formulated for oral or parenteral administration.

For oral administration, the pharmaceutical compositions may take the form of, for example, tablets or capsules prepared by conventional means with pharmaceutically acceptable excipients such as binding agents (for example pregelatinised maize starch, polyvinylpyrrolidone or hydroxypropyl methylcellulose); fillers (for example lactose, microcrystalline cellulose or calcium phosphate); lubricants (for example magnesium stearate, talc or silica); disintegrants (for example potato starch or sodium starch glycollate); or wetting agents (for example sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (for example sorbitol syrup, methyl cellulose or hydrogenated edible fats); emulsifying agents (for example lecithin or acacia); non-aqueous vehicles (for example methyl or propyl-p-hydroxybenzoates or sorbic acid).

Compounds for use according to the invention may be formulated for parenteral administration by injection conveniently intravenous or subcutaneous injection, for example by bolus injection or continuous intravenous infusion. Formulations for injection may be presented in unit dosage form, for example, in ampoules or in multi-dose containers, with an added preservative.

The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, for example sterile pyrogen-free water, before use.

The dose at which the compounds are administered for use according to the invention will depend on the particular compound used, the condition of the patient and on the frequency and route of administration. Compounds of formulae (I) to (V) disclosed in the above mentioned specifications may be administered at doses in the ranges specified therein. Thus, a unit dose will generally contain from 20 to 1000mg, preferably from 30 to 500mg which may be administered for example 1 to 4 times a day giving a total daily dose for a 70kg adult in the range of 100 to 3000mg. Alternatively, a unit dose may contain from 2 to 20mg of active ingredient and will be administered in multiples, if desired, to give the preceding daily dose.

The following compounds of formula (I) are novel and form a further feature of the invention :

1-[(3,4-Dichlorophenoxyacetyl)]-2-(1-pyrrolidinylmethyl)piperidine;

1-[(3,4-Dichlorophenyl)acetyl)]-2-[(1,2,3,6-tetrahydropyridinyl)methyl]-piperidine;

1-(4-Bromophenylacetyl)-2-(1-pyrrolidinylmethyl)piperidine;

1-[(3,4-Dichlorophenyl)acetyl)]-2-(1-piperidinylmethyl)piperidine.

Compounds of formula (I) may be prepared by the process described in the above mentioned specification. Alternatively, compounds of formula (I) may be prepared by reductive amination of a compound of formula (II)

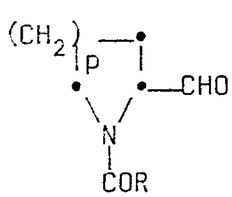

(II)

(wherein R and p are as previously defined) with an amine $R_1R_2NH$ in the presence of a suitable reducing agent.

The reduction may be affected using an alkali metal or alkaline earth metal borohydride or cyanoborohydride (for example sodium cyanoborohydride) in a suitable solvent, for example an alcohol such as methanol and at a suitable temperature, conveniently room temperature.

Compounds of formula (II) may be prepared, for example, from compounds of formula (III)

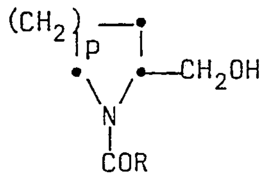

(III)

by oxidation using conventional methods, for example using an oxidising agent such as chromum trioxide in a suitable solvent, for example pyridine.

Compounds of formula (III) may be prepared from compounds of formula (IV)

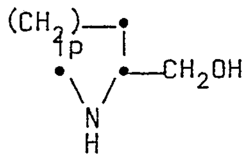

(IV)

by method analogous to those described for the preparation of compounds of formula (I) in the above mentioned specification.

Compounds of formula (IV) are known in the art.

The invention is further illustrated by the following examples. All temperatures are in °C.

Example 1

1-[(3,4-Dichlorophenoxy)acetyl]-2-(1-pyrrolidinylmethyl)piperidine fumarate (1:1)

To a solution of 3,4-dichlorophenoxyacetic acid (0.98g) in acetonitrile (20ml) was added a solution of carbonyldiimidazole (0.73g) in warm acetonitrile (10ml) followed after 10min by a solution of 2-(1-pyrrolidinylmethyl)-piperidine (0.75g) in acetonitrile (5ml). The mixture was stirred overnight at room temperature, the solvent was removed in vacuo and the residue was dissolved in dichloromethane. After washing with 2N sodium carbonate solution and water, the dried ($MgSO_4$) solvent was removed and the residual oil (1.2g) was purified by chromatography on alumina (50g. Type UGI) eluting with ether:hexane (2:1) and (3:1) to give the free base of the title compound as an oil (0.55g). The base (0.5g) was dissolved in ether and treated with a solution of fumaric acid in ethyl acetate to give the title compound as a solid, (0.61g) m.p. 177-8° decomp.

| | | | | |
|---|---|---|---|---|
| Analysis | Found: | C,54.5; | H,5.85; | N,5.45. |
| $C_{18}H_{22}Cl_2N_2O_2.C_4H_4O_4$ | requires | C,54.2; | H,5.8; | N,5.75%. |

Example 2

1-[(3,4-Dichlorophenyl)acetyl]-2-[(1,2,3,6-tetrahydropyridinyl)methyl]piperidine fumarate (1:1)

(i) 1-[(3,4-Dichlorophenyl)acetyl]-2-piperidinemethanol

A solution of 2-piperidinemethanol (0.56g) in acetonitrile (10ml) was added to a solution of 3,4-dichlorophenylacetic acid (1.02g) and 1,1′- carbonyldiimidazole (0.81g) in acetonitrile (20ml) and the mixture stirred at 22°, under nitrogen for 18h. The acetonitrile was evaporated in vacuo, the residue was dissolved in dichloromethane (50ml) and washed with 2N sodium carbonate (20ml) and then 0.5N hydrochloric acid (10ml). The organic phase was dried and evaporated in vacuo, and the residue purified by chromatography eluting with dichloromethane- methanol (9:1) to give the title compound as a crystalline solid (0.80g) m.p. 108-110°.

(ii) 1-[(3,4-Dichlorophenyl)acetyl]-2-piperidinecarboxaldehyde

Dry chromium trioxide (5.40g) was added portionwise to a solution of pyridine (9.0g, 8.6ml) in dry dichloromethane (120ml) at room temperature under nitrogen and the resulting solution was stirred for 30min. A solution of the product of stage (1) (3.0g) in dry dichloromethane (40ml) was added and the mixture stirred

for 1h. The reaction mixture was poured into ether (2L) and filtered. The filtrate was evaporated to dryness and the residue purified by flash column chromatography eluting with ethyl acetate-hexane (1:1) to give the title compound as a crystalline solid (2.05g), m.p. 64-68°.

(iii) Sodium cyanoborohydride (252mg) was added to a solution of the product of stage (ii) (0.80g) and 1,2,3,6-tetrahydropyridine (0.36ml) in methanol (20ml) in small portions. The solution was stirred at 22°, under nitrogen, for 18h. The methanol was evaporated in vacuo, the residue dissolved in dichloromethane (50ml) and washed first with 2N hydrochloric acid (2x10ml) and then 2N sodium hydroxide (1x10ml). The organic phase was dried ($Na_2SO_4$) and evaporated in vacuo. The residual oil was purified by column chromatography on alumina, eluting with ether to give an oil (0.45g). The oil was dissolved in ethyl acetate and added to a hot solution of fumaric acid in ethyl acetate. On cooling the title compound crystallised as a solid (0.32g) m.p. 181-183°.

| | | | | |
|---|---|---|---|---|
| Analysis | Found: | C,57.1; | H,5.95; | N,5.6. |
| $C_{19}H_{24}Cl_2N_2O.C_4H_4O_4$ | requires | C,57.15; | H,5.85; | N,5.8%. |

## Example 3

1-(4-Bromophenylacetyl)-2-(1-pyrrolidinylmethyl)piperidine maleate (1:1)

To a solution of 4-bromophenylacetic acid (1.28g) in acetonitrile (20ml) was added a solution of 1,1′-carbonyldiimidazole (0.96g) in warm acetontrile (10ml). After 10min a solution of 2-(1-pyrrolidinylmethyl)piperidine (1.00g) in acetonitrile (5ml) was added and the mixture stirred at ambient for 4h. The solvent was removed in vacuo and the residue partitioned between dichloromethane (50ml) and aqueous 2N sodium carbonate solution (50ml). The organic phase was separated and washed with water (100ml). The dried ($Na_2SO_4$) organic solution was evaporated and the residue was purified by column chromatography on alumina (Type UGI) eluting with ethyl acetate to give the free base of the title compound (1.58g). Maleate salt formation and crystallisation from ethyl-acetate/ethanol gave the title compound (1.86g), m.p. 144-146.5°.

| | | | | | |
|---|---|---|---|---|---|
| Analysis | Found: | C,55.25; | H,6.26; | N,5.81; | Br,16.65. |
| $C_{18}H_{21}BrNO.C_4H_4O_4$ | requires | C,54.89; | H,6.07; | N,5.82; | Br,16.60%. |

## Example 4

1-[(3,4-Dichlorophenyl)acetyl]-2-(1-piperidinylmethyl)piperidine fumarate (1:1)

Sodium cyanoborohydride (0.125g) was added in small portions to a solution of 1-[(3,4-dichlorophenyl)acetyl]-2-piperidinecarboxaldehyde (0.40g) and piperidine (0.20ml) in methanol (20ml). The solution was stirred at 22°, under nitrogen, for 18h. The methanol was evaporated in vacuo, the residual oil dissolved in dichloromethane (40ml) and washed first with 2N hydrochloric acid (2x10ml) and then 2N sodium hydroxide (10ml). The organic phase was dried ($Na_2SO_4$) and evaporated to give an oil which was dissolved in ethyl acetate and added to a solution of fumaric acid in hot ethyl acetate to give the title compound as a crystalline solid (0.18g), m.p. 178-179.5°.

| | | | | |
|---|---|---|---|---|
| Analysis | Found: | C,56.9; | H,6.25; | N,5.9. |
| $C_{19}H_{26}Cl_2N_2O.C_4H_4O_4$ | requires | C,56.9; | H,6.25; | N,5.75%. |

The following Examples illustrate pharmaceutical formulations containing 1-[(3,4-dichlorophenyl)acetyl]--2-(1-pyrrolidinylmethyl)piperidine as active ingredient for use in the treatment of cerebral ischaemia. Other compounds of formula (I) may be formulated in a similar manner.

TABLETS FOR ORAL ADMINISTRATION

DIRECT COMPRESSION

| | mg/tablet |
|---|---|
| Active ingredient | 20 |
| Calcium Hydrogen Phosphate B.P. * | 75.5 |
| Croscarmellose sodium USP | 4 |
| Magnesium Stearate, B.P. | 0.5 |
| Compression weight | 100mg |

* of a grade suitable for direct compression

The active ingredient is sieved before use. The calcium hydrogen phosphate, croscarmellose sodium and active ingredient are weighed into a clean polythene bag. The powders are mixed by vigorous shaking then the magnesium stearate is weighed and added to the mix which is blended further. The mix is then compressed using a Manesty F3 tablet machine fitted with 5.5mm flat bevelled edge punches, into tablets with target compression weight of 100mg.

Tablets may also be prepared by other conventional methods such as wet granulation.

Tablets of other strengths may be prepared by altering the ratio of active ingredient to lactose or the compression weight and using punches to suit.

The tablets may be film coated with suitable film forming materials, such as hydroxypropyl methylcellulose, using standard techniques. Alternatively the tablets may be sugar coated.

INJECTION FOR INTRAVENOUS ADMINISTRATION

| | mg/ml |
|---|---|
| Active ingredient | 5 |
| Sodium Chloride BP | as required |
| Water for Injection BP 0.5 to 2mℓ | |

INTRAVENOUS INFUSION

| | |
|---|---|
| Dextrose 5% aqueous solution BP | 10-100ml |
| Active ingredient | 700mg |
| Sodium chloride BP | as required |

For infusion at a rate of 700mg per hour.

Sodium chloride may be added to adjust the tonicity of the solution and the pH may be adjusted, using acid or alkali, to that of optimum stability and/or to facilitate solution of the active ingredient. Alternatively suitable buffer salts may be used.

The solution is prepared, clarified and filled into appropriate size ampoules sealed by fusion of the glass. The injection is sterilised by heating in an autoclave using one of the acceptable cycles. Alternatively the solution may be sterilised by filtration and filled into sterile ampoules under aseptic conditions. The solution may be packed under an inert atmosphere of nitrogen or other suitable gas.

**Claims**

1. Use of a compound or a pharmaceutically acceptable salt or solvate thereof for the manufacture of a medicament for the treatment of cerebral ischaemia wherein the compound is selected from a compound of formula (I), (II), (III), (IV) or (V)

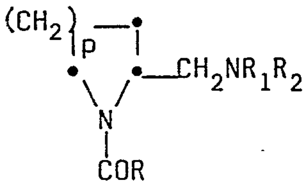

(I)

wherein
RCO represents an acyl group containing a substituted or unsubstituted carbocyclic or heterocyclic aromatic ring;
$R_1$ and $R_2$ are independently $C_{1-6}$ alkyl groups or together form a $C_{3-6}$ polymethylene or alkenylene group;
and p is 1, 2, 3 or 4;

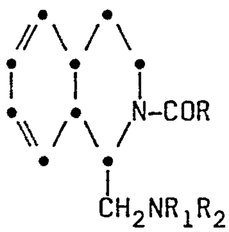

(II)

wherein R, $R_1$ and $R_2$ are as defined for formula (I);

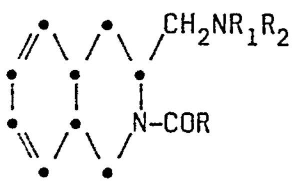

(III)

wherein R, $R_1$ and $R_2$ are as defined for formula (I);

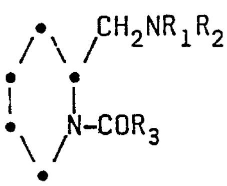

(IV)

wherein
$R_3CO$ is an acyl group in which $R_3$ represents

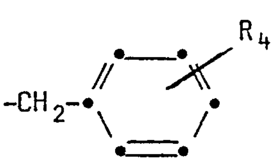

and
$R_4$ is Br, $NO_2$ or $CF_3$;
and $R_1$ and $R_2$ are as defined for formula (I);

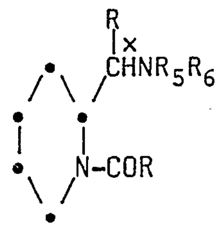

wherein
R is as defined for formula (I);
$R_5$ and $R_6$ are independently hydrogen, $C_{1-6}$alkyl, $C_{1-6}$alkenyl $C_{1-6}$cycloalkyl or $C_{1-12}$cycloalkylalkyl or together form a $C_{2-6}$polymethylene or $C_{2-6}$alkenylene group optionally substituted with a heteroatom, provided that $R_5$ and $R_6$ do not both represent hydrogen;
$R_x$ is $C_{1-6}$ alkyl or phenyl, or $R_x$ together with $R_5$ form a $-(CH_2)3-$or $-(CH_2)_4-$ group.

2. A compound selected from :
1-[(3,4-Dichlorophenoxyacetyl)]-2-(1-pyrrolidinylmethyl)piperidine;
1-[(3,4-Dichlorophenyl)acetyl)]-2-[(1,2,3,6-tetrahydropyridinyl)methyl]piperidine;
1-(4-Bromophenylacetyl)-2-(1-pyrrolidinylmethyl)piperidine;
1-[(3,4-Dichlorophenyl)acetyl)]-2-(1-piperidinylmethyl)piperidine;

3. Use of a compound in the manufacture of a medicament according to Claim 1 wherein the compound is as defined in Claim 2.